(19) Europäisches Patentamt
European Patent Office
Office européen des brevets

(11) EP 1 145 716 B1

(12) EUROPEAN PATENT SPECIFICATION

(45) Date of publication and mention of the grant of the patent:
**04.08.2004 Bulletin 2004/32**

(51) Int Cl.7: **A61K 31/519**, A61P 17/00

(21) Application number: **01109332.5**

(22) Date of filing: **12.04.2001**

# (54) Therapeutic agent for the treatment of dermatitis

Therapeutisches Mittel zur Behandlung von Dermatitis

Agent thérapeutique pour traitement de la dermatite

(84) Designated Contracting States:
**AT BE CH CY DE DK ES FI FR GB GR IE IT LI LU MC NL PT SE TR**

(30) Priority: **13.04.2000 JP 2000111809**

(43) Date of publication of application:
**17.10.2001 Bulletin 2001/42**

(73) Proprietor: **NIPPON ZOKI PHARMACEUTICAL CO., LTD.**
**Chuo-ku, Osaka (JP)**

(72) Inventors:
- **Yoshii, Haruo, Inst. of Bio-Active Science Yashiro-cho, Katoh-gun, Hyogo (JP)**
- **Fujita, Akihiro, Inst. of Bio-Active Science Yashiro-cho, Katoh-gun, Hyogo (JP)**

(74) Representative: **HOFFMANN - EITLE**
**Patent- und Rechtsanwälte**
**Arabellastrasse 4**
**81925 München (DE)**

(56) References cited:
**EP-A- 0 243 311**
**EP-A- 0 994 113**
**WO-A-95/01980**

- **HANIFIN J M ET AL: "TYPE 4 PHOSPHODIESTERASE INHIBITORS HAVE CLINICAL AND IN VITRO ANTI-INFLAMMATORY EFFECTS IN ATOPIC DERMATITIS" JOURNAL OF INVESTIGATIVE DERMATOLOGY, NEW YORK, NY, US, vol. 107, no. 1, 1996, pages 51-56, XP000915565 ISSN: 0022-202X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 145 716 B1

## Description

Detailed Description of the Invention:

[Technical Field of the Invention]

The present invention relates to a medical use of 7-amino-3-benzyl-1-phenylpyrido[2,3-d]pyrimidin-2,4-dione derivatives as a therapeutic agent for dermatitis, particularly as a therapeutic agent for atopic dermatitis.

[Prior Art]

Dermatitis is an inflammation reaction of the skin to various external and internal causes, is the most popular disease among the skin diseases and, usually, has the same meaning as eczema. Typical clinical feature in the acute stage is that swelling erythema is formed, then papule and serous papule are formed on the erythema and, after formation of vesicle, pustule, erosion, crust and desquamation, the disease becomes to healing. When it turns chronic, thickening, lichenification and pigmentation of the skin are resulted and, in most cases, itching is accompanied therewith. Histologically it is characterized in swelling among epidermal cells (in a spongy state) during the acute stage. Contact dermatitis, atopic dermatitis, seborrheic dermatitis, nummular eczema, Vidal lichen, stasis dermatitis, dyshidrotic eczema, asteatosis eczema dermatitis, autosensitization eczema, etc. are included within a category of dermatitis.

Although atopic dermatitis is thought to occur by atopy, i.e. by an allergic reaction in which immunoglobulin E (IgE) is participated, it is still ambiguous even at present for a definite cause of its onset. Atopic dermatitis is often accompanied by a high level of IgE in blood and eosinophilia. Atopy itself belongs to type I but, since atopic dermatitis is a reaction similar to eczema and contact dermatitis in a pathological view, participation of allergy of type IV (delayed type) has been suggested. It is also suggested that a delayed type reaction accompanied by infiltration of eosinophils and lymphocytes plays an important role for onset of atopic dermatitis and its change to chronic one (refer to an article by Iwamoto, et al.: *J. Leukoc. Biol.*, 52, pages 572-578 (1992); an article hy Frigas, et al.: *J. Allergy Clin. Immunol.*, 77, pages 527-537 (1986), etc.). Symptom of atopic dermatitis is that, from about second to sixth months from birth, weeping eczema of the face is firstly noted and then it gradually expands to the legs and arms and to the trunk. A strong itching is also characteristic. The symptom changes with the growing and is classified into a baby period (younger than 2 years age), an infant period (2-12 years age) and an adult period. During a baby period, the onset is mostly limited to the face but, gradually, skin of trunk becomes dry resulting in follicular papule (atopic skin). In the period of infant and small children, the lesion becomes dry and thick area is formed in pits of elbow and knee (lichenification). There are many cases where it heals by about 12 years age but, when it is carried over to an adult period, it becomes severer and, even if once turns light, recurrence and worsening are often noted and there are even some cases that a long period is needed until a complete healing or a complete healing is not achieved. With regard to the therapy, application of a steroidal agent for external use (ointment or the like) is most effective and any therapeutic method in place of that has not been established yet.

Incidentally, steroid preparations which are frequently used for the therapy of dermatitis including atopic dermatitis are the pharmaceuticals which have very active clinical effect and also show a great variety of adverse reactions. There have been various reports on the side effects of steroid preparations and, in the case of agents for external use such as ointment, direct harmful effects to the skin such as thinning, shrink and flushing of the skin have been becoming a social problem as severe adverse reactions. As such, there is a problem of severe adverse reaction in the steroid preparations which have been frequently used as remedies for dermatitis and atopic dermatitis whereby there has been a eager demand in patients and in medical field for the pharmaceuticals which have less side effect and higher safety being able to be used without risk.

For the compounds which are used as a therapeutic agent for dermatitis according to the present invention, a patent application has been filed already (Japanese Patent Laid-Open 2000-119272: U.S. Patent Application S.N. 09/418982). With regard to the compounds having the similar structure to the above, it has been also reported already that the compounds having a pyrido[2,3-a]pyrimidine structure have an anti-allergic action (Japanese Patent Laid-Open Sho-63/45279; USP 4808587) and that the compound having a 7-aminopyrido[2,3-d]pyrimidine structure have a bronchodilating action (Japanese Patent Laid-Open Hei-8/3046, Hei-8/3164 and Hei-8/3165; USP 5776942).

[Problems to be Solved by the Invention]

The present invention is to solve the above-mentioned problems in the prior art and is to provide a therapeutic agent for dermatitis, particularly a therapeutic agent for atopic dermatitis, showing little adverse reaction and having high safety which has been eagerly demanded by patients and in the medical field.

[Means for Solving the Problems]

The present inventors have carried out an intensive investigation for 7-aminopyrido[2,3-d]pyrimidine derivatives and, as a result, they have found that 7-amino-3-benzyl-1-phenylpyrido[2,3-d]pyrimidin-2,4-dione derivatives are useful as a therapeutic agent for dermatitis, particularly for atopic dermatitis, and have little adverse reaction to the skin whereupon the present invention has been achieved.

[Preferred Embodiments of the Invention]

The present invention relates to a therapeutic agent for dermatitis containing a compound represented by the following formula (I) or a pharmaceutically acceptable salt or hydrate thereof as an effective ingredient.

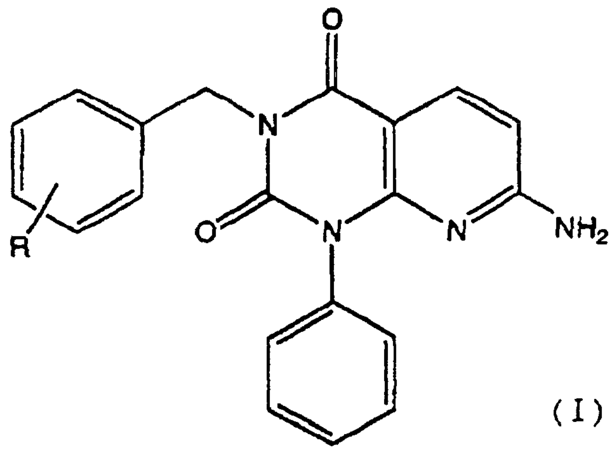

(I)

(In the formula, R is hydrogen or halogen.)

In the substituent in the above-mentioned formula (I), R is hydrogen or halogen such as fluorine, chlorine, bromine or iodine.

The preferred embodiments of the present invention are as follows:

(1) A therapeutic agent for dermatitis containing a compound represented by the above formula (I) or a pharmaceutically acceptable salt or hydrate thereof as an effective ingredient.
(2) The therapeutic agent for dermatitis according to (1), wherein the agent is a therapeutic agent for atopic dermatitis.
(3) The therapeutic agent for dermatitis according to (1) or (2), wherein the agent is that for external use.
(4) The therapeutic agent for dermatitis according to any of (1) to (3), wherein R is hydrogen in the formula (I).
(5) The therapeutic agent for dermatitis according to any of (1) to (3), wherein R is substituted at o-position in the formula (I).
(6) The therapeutic agent for dermatitis according to (5), wherein R is chloride.

Among the compounds used for the therapeutic agent for dermatitis of the present invention, more preferred compounds are as follows.

7-amino-3-benzyl-1,2,3,4-tetrahydro-1-phenylpyrido[2,3-d]pyrimidin-2,4-dione [compound 1]
7-amino-3-(2-chlorobenzyl)-1,2,3,4-tetrahydro-1-phenylpyrido[2,3-d]p yrimidin-2,4-dione [compound 2]

The most preferred compound is the compound 1.

The compounds represented by the above formula (I) used as a therapeutic agent for dermatitis according to the present invention can be manufactured by a method mentioned in the specifications of Japanese Patent Laid-Open Sho-63/45279 (USP 4808587, EP 0243311 B), Hei-8/3049, Hei-8/3164, Hei-8/3165 (USP 5776942, EP 0696590 A) and 2000-119272 (US SN 09/418982, EP 0994113 A) or by a similar method thereto and the said manufacturing method will be mentioned in Examples later.

The compounds represented by the above formula (I) include the pharmaceutically acceptable salts of thereof

such as acid addition salts with hydrochloric acid, sulfuric acid, nitric acid, hydrobromic acid, phosphoric acid, perchloric acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid or sulfanilic acid; salts with alkali metal such as sodium or potassium, salts with alkaline-earth metal such as calcium or magnesium, or salts with other metals such as aluminum: or salts with bases such as ammonia or organic amines. Those salts may be manufactured by known methods from the compounds of the present invention in a free state or may be mutually converted among the salts. When there are steric isomers such as cis-trans isomer, optical isomer, conformational isomer and hydrate for the substances of the present invention, the present invention includes any and all of them.

The compound represented by the above formula (I) is combined with an appropriate pharmaceutical carrier or diluent whereupon a pharmaceutical agent which is used as a therapeutic agent for dermatitis according to the present invention can be prepared. With regard to a method for the manufacture of the preparation, any of conventional methods may be used. As a therapeutic agent for dermatitis, it is optimum to manufacture a preparation for external use such as liquid, suspension/emulsion, plaster, ointment, cataplasm, liniment and lotion. For prescription, the compound of the above formula (I) may be used in a form of its pharmaceutically acceptable salt or hydrate, or may be conbined with other pharmaceutically active ingredient(s). Method for the manufacture of the preparation for external use is mentioned in detail, for example, in General Rule for Pharmaceutical Preparations, Commentary to the 13th Revision of the Japanese Pharmacopoeia (published by Hirokawa Shoten, 1996).

Ointment preparation may be roughly classified into fat/oil type ointment, emulsified ointment, water-soluble ointment and suspended ointment according to the type of the base (vehicle) used therefor. Manufacture of such an ointment may be carried out, for example, using fat, fatty oil, lanolin, vaseline, paraffin, wax, resin, plastics, glycols, higher alcohol, glycerol, water, emulsifier, suspending agent or other appropriate additive as a material or as a vehicle and by adding the compound of the present invention followed by mixing to make the mixture homogeneous.

For the manufacture of cataplasm, powder of the compound of the above formula (I) is mixed with an essential oil component to give a muddy product. It is also possible, depending upon the type and state of the disease, to manufacture other types of preparation which is optimum for the therapy.

The preferred dose of the compound of the present invention may vary depending upon the object to be administered the patient, form of the preparation, method for the administration, term for the administration, etc. For example, in order to achieve a desired effect, ointment containing 0.1% to 15% of the compound of the present invention may be applied on the affected part once to several times per day.

The present invention will be further illustrated by way of the following examples although the present invention is not limited by them at all.

Examples

The starting materials may be purchased from Aldrich Chemical Co., Inc.; Furuka Chemical Inc.; Lancaster Synthesis Inc.; Maybridge Chemical Co., Ltd.; or Tokyo Kasei K.K. or may be synthesized by known methods mentioned in literatures such as *J. Org. Chem.,* 16, 1879 (1951); *J. Am. Chem. Soc*., **75,** 114 (1953); etc.

Example 1. Preparation of Ointment.

(1) Manufacture of 6-Amino-1-phenyluracil.

Phenylurea (68.1 g, 0.5 mol) and ethyl cyanoacetate (53 mL, 0.5 mol) were added to a solution prepared by mixing methanol (400 mL) with potassium tert-butoxide (67.3 g, 0.6 mol). The mixture was heated to reflux for 5.5 hours, the solvent was evaporated *in vacuo* until a residue was formed and the residue was dissolved in hot water (2 L). Glacial acetic acid was added to the solution until the solution became acidic and a uracil derivative was obtained as a bulky and yellowish precipitate. The precipitate was collected by filtration, washed for several times with water and dried at 50°C to give 6-amino-1-phenyluracil (67.4 g) in a yield of 66%. Mp >280°C
$^1$H-NMR (DMSO-$d_6$) δ: 4.67(s,1H), 6.08(s,2H), 7.31(d,2H,J=7Hz),
7.47-7.54(m,3H), 10.43(s,1H)
IR (KBr) : 3478, 3334, 2981, 1712, 1634, 1476, 1387, 1299, 704 $cm^{-1}$
MS (EI) m/z : 203[$M^+$], 160, 132, 77

(2) Manufacture of 7-Amino-1,2,3,4-tetrahydro-1-phenylpyrido[2,3-d]pyrimidin-2,4-dione.

6-Amino-1-phenyluracil (30.5 g, 150 mmol) and propene 3-methoxycyanide (25 mL, 225 mmol) were added to a solution prepared by mixing tert-butanol (300 mL) with potassium tert-butoxide (18.7 g. 225 mmol). The mixture

was heated at 110°C for 12 hours with stirring. Butanol was evaporated, water (200 mL) was added to the residue and the mixture was acidified with acetic acid. The precipitate was collected by filtration and washed with hot ethanol to give 7-amino-1,2,3,4-tetrahydro-1-phenylpyrido[2,3-d]pyrimidin-2,4-dione (20 g) in a yield of 53%.
Mp >300°C
$^1$H-NMR (DMSO-$d_6$)δ: 6.27(d,1H,J=9 Hz), 6.89(s,2H), 7.20-7.87(m,5H), 7.88(d,1H,J=9Hz), 11.26(s,1H)
IR (KBr) : 3038, 1709, 1605, 1417, 789 $cm^{-1}$
MS (EI) m/z : 254[$M^+$], 211, 77

(3) Manufacture of 7-amino-1,2,3,4-tetrahydro-1-phenylpyrido[2,3-d]pyrimidin-2,4-dione [compound 1].

A suspension prepared by adding 7-amino-3-benyzl-1,2,3,4-tetrahydro-1-phenylpyrido[2,3-d]pyrimidin-2,4-dio ne (3 g, 12 mmol) and ammonium sulfate (500 mg) to hexamethyldisilazane (100 mL) was refluxed at room temperature and atmospheric pressure for 24 hours. The solvent was evaporated and the residue was dissolved in 100 mL of L(-)-5,6,7,8-tetrahydrofolic acid (THF). Into this solution was dropped a solution prepared by adding benzyl bromide (2 mL, 18 mmol) and 1M Bu4NF to THF (14 mL. 14 mmol) under refluxing. The mixed solution was stirred for 2 hours and concentrated *in vacuo*. The residue was purified by a silica gel column chromatography (benzene:acetone = 5:1) and recrystallized from methanol to give 7-amino-3-benyzl-1,2,3,4-tetrahydro-1-phenylpyrido[2,3-d]pyrimidin-2,4-dio ne [compound 1] (1 g) in a yield of 27%.
Mp 261-262 °C
$^1$H-NMR (DMSO-$d_6$)δ: 5.08(s,2H), 6.32(d,1H,J=8 Hz), 6.98(s,2H),
7.22-7.49(m,10H), 7.94(d,1H,J=8 Hz)
IR (KBr) : 3498, 3392, 1695, 1655, 1618, 787, 700 $cm^{-1}$
Anal. Calcd for $C_{20}H_{16}N_4O_2$ : C, 69.76; H, 4.68; N, 16.27,
Found : C, 69.81; H, 4.77; N, 16.13
MS (EI) m/z : 344[$M^+$], 211. 91

7-amino-3-(2-chlorobenzyl)-1,2,3,4-tetrahydro-1-phenylpyrido[2,3·d]p yrimidin-2,4-dione [compound 2] was also prepared by utilizing the above methods of (1) to (3).

(4) The compound 1 or 2 prepared as above was well suspended in white vaseline in a mortar to prepare an ointment where the final concentration was 0.3%, 1%, 3% or 10%. As a control, an ointment consisting of a base only was prepared and, as a positive control, an ointment containing 0.12% of betamethasone valerate (a steroid preparation) was prepared.

Example 2. Preparation of Animal Model for Atopic Dermatitis.

BALB/c female (SPF) mice of seven weeks age were purchased and subjected to an experiment. According to a method of Tanaka, et al. (*Allergy*, 46, pages 42-48(1997)), egg white albumin (final concentration: 2 μg/mL) and aluminum hydroxide gel (final concentration: 10 mg/mL) were suspended in a physiological saline, 0.5 mL of the suspension was subjected to an intraperitoneal sensitization on the first day of the test and, on the 14th day, an additional sensitization was carried out under the same condition. Further, on the 28th day, 25 μL of egg albumin (a 20 μg/mL solution in a physiological saline) was intradermally administered to the right ear of a mouse to induce a swelling reaction.

This swelling reaction reached its peak at 4 hours after the challenge by antigen and, even after 24 to 48 hours, a significantly high swelling reaction was noted as compared with the control in which antigen was merely challenged (not sensitized). In this model, the reaction at 1-4 hour(s) after the challenge is thought to be a swelling reaction of an immediate type caused by various mediators from mast cells and it seems that there is almost no participation of inflammation cells such as eosinophils and lymphocytes. On the contrary, the swelling from 24 to 48 hours after the challenge is thought to be the so-called delayed-type swelling reaction accompanied by infiltration of eosinophils, neutrophils and lymphocytes. In addition, appearance of flare reaction having a peak at 24th hours after the challenge was noted on the ear of the antigen-induced site. As same a swelling, flare is one of the important symptoms of inflammation. Although the occurrence mechanism of the flare reaction is ambiguous, it may be a flare reaction of a delayed type in view of time course of the expression. As such, this model animal where the inflammation of both immediate and delayed types are induced is appropriate as a model animal for atopic dermatitis. In the following experiment, action of the therapeutic agent for dermatitis according to the present invention was tested using the said model animal.

In the following Examples 3 to 6, there are shown the results of the investigation on the action and safety of the therapeutic agent for dermatitis according to the present invention. Incidentally, with regard to the statistical treatment in the following Examples, the result is given by a mean value ± standard deviation and a statistical library (Yukms) was used for the test between the groups where a Student's two-sided unpaired t-test was carried out for an analysis

between the vehicle-only control group and the challenge-only group or the untreated group while Dunnett's both-sided multiple comparison test or Mann-Whitney test was carried out between the control group and the test drug group.

Example 3. Suppressive Action to Antigen-Induced Swelling Reaction in Ear of Mouse.

Each of a vehicle (control) and a test agent (containing the compound of the present invention or positive control) was applied to the antigen-induced site (right auricle) of mouse where a swelling reaction was induced in accordance with Example 2 at 2 hours before and 4 hours after the challenge. After the challenge, thickness of the induced ear was measured by a Dial Thickness Gauge (manufactured by Ozaki Seisakusho). Intensity of the reaction was expressed in terms of an increase in the auricle thickness by deducting the thickness (early value) measured before the challenge from the thickness of the induced ear thickness. The suppressing rate of the test drug for ear swelling was calculated by the following formula.

Suppressing Rate (%) = 100 × [(Ear Thickness Increase of Vehicle-Only-Control) - (Ear Thickness Increase of the Test Group)]/[(Ear Thickness Increase of the Vehicle·Only·Control) - (Ear Thickness Increase of the Challenge-Only-Group)]

Examples of the results are shown in Tables 1 and 2. The therapeutic agent for dermatitis according to the present invention significantly and dose-dependently suppressed the antigen-induced swelling reaction of mouse ear.

Table 1

| | n | Ear Thickness Increase at 1 hr after Challenge × 0.01 mm Suppressing Rate | Ear Thickness Increase at 4 hr after Challenge × 0.01 mm Suppressing Rate | Ear Thickness Increase at 8 hr after Challenge × 0.01 mm Suppressing Rate | Ear Thickness Increase at 24 hr after Challenge × 0.01 mm Suppressing Rate | Ear Thickness Increase at 48 hr after Challenge × 0.01 mm Suppressing Rate |
|---|---|---|---|---|---|---|
| Challenge Only | 2 | 6.5 ± 0.5# | 4.0 ± 1.0### | 3.5 ± 1.5### | 2.0 ± 2.0### | 2.0 ± 2.0### |
| No-Treatment-Control | 5 | 9.0 ± 1.1 | 25.2 ± 0.9 | 19.2 ± 0.6 | 14.0 ± 1.5 | 9.2 ± 1.6 |
| Vehicle-Only-Control | 5 | 9.8 ± 0.6 | 25.2 ± 0.6 | 20.4 ± 1.1 | 17.0 ± 1.0 | 11.2 ± 09 |
| Positive Control (Steroid Preparation, 0.12% Ointment) | 3 | 8.3 ± 07 (45.5 %) | 16.3 ± 2.0** (42.0 %) | 12.0 ± 1.2**** (49.7 %) | 5.0 ± 1.0** (80.0 %) | 2.0 ± 1.0** (100.0 %) |
| Compound 1 (1% Ointment) | 4 | 6.3 ± 1.4* (106.1 %) | 18.0 ± 0.9**** (34.0 %) | 16.0 ± 0.4* (26.0 %) | 9.3 ± 1.1** (51.3 %) | 6.8 ± 1.0 (47.8 %) |
| Compound 1 (3% Ointment) | 4 | 5.3 ± 0.5** (136.4 %) | 21.3 ± 0.9* (18.4 %) | 15.8 ± 1.4* (27.2 %) | 7.8 ± 0.9** (61.3 %) | 5.3 ± 0.9* (64.1 %) |

Average ± Standard Error
Significant Difference from Base-Only-Control:
*P<0.05,
**P<0.01 (Dunnett Multiple Comparison)
#P<0.05,
###P<0.001 (Student's t-Test)

Table 2

| | n | Ear Thickness Increase at 4 hr after Challenge × 0.01 mm Suppressing Rate | Ear Thickness Increase at 24 hr after Challenge × 0.01 mm Suppressing Rate |
|---|---|---|---|
| Challenge Only | 1 | 2.0 | 1.0 |
| Vehicle-Only-Control | 4 | 24.5 ± 1.7 | 16.5 ± 1.6 |
| Positive Control (Steroid Preparation, 0.12% Ointment) | 4 | 11.0 ± 0.6** (60.0 %) | 6.3 ± 2.4* (65.8 %) |
| Compound 1 (3% Ointment) | 4 | 13.3 ± 0.3** (49.8 %) | 9.3 ± 1.7 (46.5 %) |
| Compound 1 (0.3% Ointment) | 4 | 20.3 ± 2.4 (18.7 %) | 13.5 ± 1.3 (19.4 %) |
| Compound 2 (1% Ointment) | 4 | 19.5 ± 3.2 (22.2 %) | 11.8 ± 2.6 (30.3 %) |
| Compound 2 (3% Ointment) | 4 | 19.3 ± 2.4 (23.1 %) | 8.0 ±1.4* (54.8 %) |

Average ± Standard Error
Significant Difference from Base-Only-Control:
*P<0.05,
**P<0.01 (Dunnett Multiple Comparison)

Example 4. Suppressive Action to Antigen-Induced Flare Reaction of Mouse.

Each of a vehicle (control) and an agent for the test (the therapeutic agent for dermatitis according to the present invention; positive control) was applied to the antigen-induced site (right auricle) of mouse where a swelling reaction was induced in accordance with Example 2 at 2 hours before and 4 hours after the challenge. The right ear was observed by naked eye and the degree of flare was scored according to the following evaluating standard.

0: no flare
1:in such a degree that flare was confirmed, the color is clear red or pale red and the size was small
2: between score 3 and score 1
3: color of the flare was clear red and the size was big as well

An example of the result is shown in Table 3. The therapeutic agent for dermatitis according to the present invention significantly suppressed the antigen-induced flare reaction of mouse.

Table 3

| | | Flare Score at 4 hr after Challenge | Flare Score at 24 hr after Challenge |
|---|---|---|---|
| | n | Suppressing Rate | Suppressing Rate |
| Vehicle-Only-Control | 23 | 0.8 ± 0 | 2.5 ± 0.2 |
| Positive Control (Steroid Preparation, 0.12% Ointment) | 20 | 0.6 ± 0 (25.0 %) | 1.5 ± 0.2*** (40.0 %) |
| Compound 1 (3% Ointment) | 22 | 0.8 ± 0 (0.0 %) | 1.7 ± 0.2* (32.0 %) |

Average ± Standard Error
Significant Difference from Base-Only-Control:
*P<0.05,
***P<0.001 (Mann-Whitney test)

Example 5. Suppressive Action to Eosinophil Infiltration to Antigen-Induced Ear of Mouse.

Degree of eosinophil infiltration to ear of mouse was measured using an eosinophil peroxidase activity in a skin tissue homogenate as an index. Each of the vehicle (control) and the test drug (the therapeutic agent for dermatitis according to the present invention; positive control) was applied to the antigen-induced site (right auricle) where swelling reaction was induced according to Example 2 at 2 hours before and 4 hours after the challenge. Afcer 24 hours from the challenge, right ear was cut, ear tissues in a group were pooled in one and a homogenate of the ear tissues was prepared according to a method of Holliday, et al. (*Toxicology*, 106, pages 237-242 (1996)). This homogenate was centrifuged at 20,000 rpm for 20 minutes (using KR-20000T, Kubota) and the peroxidase activity in the supernatant fluid was measured using o-phenylenediamine dihydrochloride (Sigma) as a substrate. The measurement was carried out by measuring the absorbance at 492 nm, evaluation was made within such a range that the reaction curve was in a linear relation and the degree of eosinophil infiltration was compared wherein that of the control was defined as 100%.

The therapeutic agent for dermatitis according to the present invention suppressed the eosinophil infiltration to antigen-induced ear of mouse in a dose-dependent manner. The eosinophil infiltration suppressing action of a 3% ointment of the compound 1 was in the same degree as that of the steroid preparation of the positive control.

Example 6. Influence of Repeated Application to Ear Thickness of Normal Mouse.

The therapeutic agent for dermatitis according to the present invention or the positive control (steroid preparation) was applied twice daily to the right ear of normal mouse for nine times in total and, after 2 hours from the final application, thickness of the ear was measured.

An examples of the result is shown in Table 2. As shown in the above test result, the therapeutic agent for dermatitis according to the present invention showed excellent ear swelling suppressive action, flare suppressive action and eosinophil infiltration suppressive action in its 3% ointment and, even by a repeated application of a 10% ointment which was considerably in a higher concentration than the above, no influence on the skin was noted at all. However, the repeated application of a positive control (steroid preparation) significantly reduced the auricle thickness.

Table 4

| | n | Ear Thickness × 0.01 mm |
|---|---|---|
| No Treatment Vehicle-Only-Control | 6 | 22.2 ± 0.4 |
| Positive Control (Steroid Preparation, 0.12% Ointment) | 6 | 18.3 ± 0.5** |
| Compound 1 (3% Ointment) | 6 | 22.3±0.2 |
| Compound 1 (10% Ointment) | 6 | 22.3 ± 0.5 |

Average ± Standard Error
Significant Difference from Base-Only-Control:
**P<0.01 (Dunnett Multiple Comparison)

Example 7. Suppressive Action to Chemotaxis of Guinea Pig Eosinophils in Abdominal Cavity.

The suppressive effect of the compounds of the present invention on the $LTB_4$ induced chemotaxis of guinea pig eosinophils in abdominal cavity were investigated using a modified Boyden chamber method. The guinea pig eosinophils in abdominal cavity were prepared using Hartley male guinea pigs according to the conventional method. The chemotaxis of the eosinophils was induced by $10^{-7}$ M of $LTB_4$ in 37 °C for 2 hours in the presence or absence of the test drug (the therapeutic agent for dermatitis of the present invention). After chemotaxis cells were fixed and stained using the Giemsa stain, the number of chemotaxis cells was counted in10 visual fields on microscope (x 400). The experiments were repeated several times and the suppressing rate of the test drug was calculated by the following formula. The 50 % inhibitory concentration ($IC_{50}$) was calculated from the regression line obtained by the least square method based on the calculated suppressing rates.

Suppressing Rate (%) = 100 × [(the number of chemotaxis

eosinophils in control group) - (the number of chemotaxis eosinophils in the

test drug group)]/[(the number of chemotaxis eosinophils in control group) -

(the number of spontaneous chemotaxis eosinophils)]

As a result of 5 times experiments using Compound 1, it showed remarkable suppressive action to eosinophil chemotaxis ($IC_{50}$ of Compound 1: 1.9 ± 0.4 μmol/L).

Example 8. Inhibitory Action to Phosphodiesterase.

The inhibitory action of the compounds of the present invention on activities of various phosphodiesterase (PDE) isozymes were investigated using the conventional method. Each of inhibitory action of the compounds of the present invention on activities of phosphodiesterase type II (PDE II) partially purified from human platelets, phosphodiesterase type III (PDE III) partially purified from human platelets, phosphodiesterase type IV (PDE IV) partially purified from human U937 promonocytic leukemia cells and phosphodiesterase type V (PDE V) partially purified from human platelets were measured.

The inhibitory ratio in the presence of 100 μM of the present compound 1 on activities of PDE II, III, IV and V were 6%, 22%, 82% and 15%, respectively. As shown above, they clearly indicated the PDE IV selective inhibitory effect. Furthermore, this PDE IV inhibitory effect was dose dependently.

[Advantage of the Invention]

As shown in the above animal experiment, the 7-amino-3-benzyl-1-phenylpyrido[2,3-d]pyrimidin-2,4-dione derivatives significantly and dose-dependently suppressed the antigen-induced auricle swelling reaction in mouse which was an atopic dermatitis model and also significantly suppressed the antigen-induced flare reaction in mouse accompanied therewith. They also had a suppressive action to eosinophil infiltration to antigen-induced auricle of mouse. Accordingly, the 7-amino-3-benzyl-1-phenylpyrido[2,3-d]pyrimidin-2,4-dione derivatives of the present invention are useful as a therapeutic agent for dermatitis such as atopic dermatitis, eczema and contact dermatitis.

Further, a direct harmful action (adverse reaction) to the skin such as thinning of the skin noted in the case of steroid preparations was not noted at all in the therapeutic agent for dermatitis according to the present invention and the said agent had a very high safety.

As mentioned hereinabove, the therapeutic agent for dermatitis according to the present invention is useful as a therapeutic agent for atopic dermatitis and dermatitis, is a pharmaceutical agent having little adverse action and high safety solving the technical problems in the prior art and is highly useful as an agent which has been briskly demanded from patients and medical field.

## Claims

1. Use of a compound represented by the following formula (I)

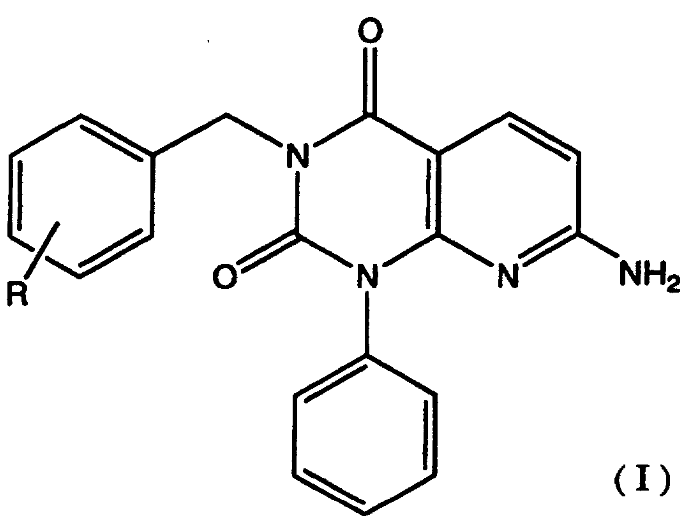

(I)

wherein R is hydrogen or halogen, or a pharmaceutically acceptable salt or hydrate thereof for the preparation of a medicament for the treatment of dermatitis.

2. Use according to claim 1, wherein R in formula (I) is hydrogen or chlorine.

3. Use according to any one of claims 1 or 2, wherein the substituent R is in o-position.

4. Use according to any one of claims 1 to 3, wherein the dermatitis is atopic dermatitis.

5. Use according to any one of claims 1 to 4, wherein the medicament is for external use.

**Patentansprüche**

1. Verwendung einer Verbindung der folgenden Formel (I):

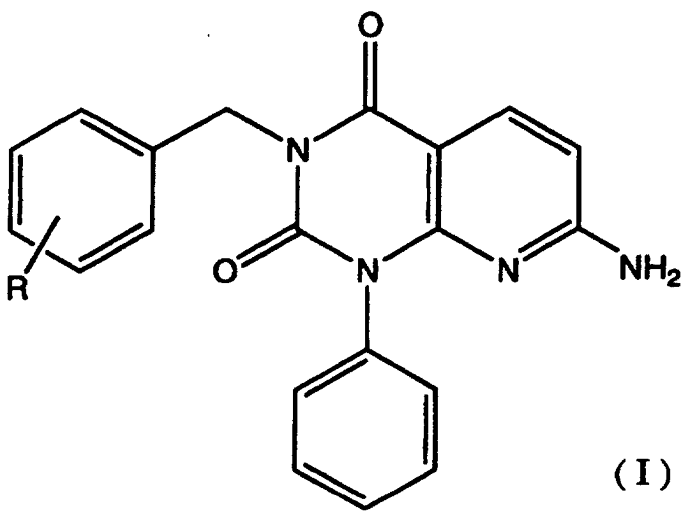

(I)

worin R Wasserstoff oder Halogen ist, oder eines pharmazeutisch annehmbaren Salzes oder Hydrats davon, zur Herstellung eines Medikaments zur Behandlung von Dermatitis.

2. Verwendung gemäss Anspruch 1, worin R in Formel (I) Wasserstoff oder Chlor ist.

3. Verwendung gemäss mindestens einem der Ansprüche 1 oder 2, worin der Substituent R in der o-Position befindlich ist.

4. Verwendung gemäss mindestens einem der Ansprüche 1 bis 3, worin die Dermatitis atopische Dermatitis ist.

5. Verwendung gemäss mindestens einem der Ansprüche 1 bis 4, worin das Medikament zur äusserlichen Anwendung bestimmt ist.

**Revendications**

1. Utilisation d'un composé représenté par la formule suivante (I)

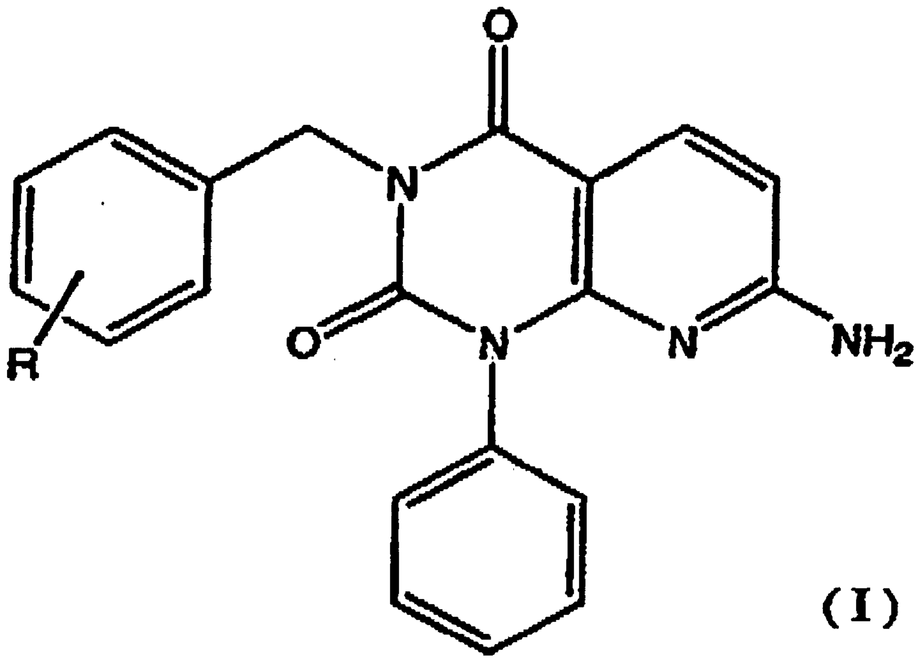

(I)

où R est un hydrogène ou un halogène, ou un de ses sels ou un de ses hydrates acceptable d'un point de vue pharmaceutique pour la préparation d'un médicament pour le traitement de la dermatite.

2. Utilisation selon la revendication 1, dans laquelle R dans la formule (I) est un hydrogène ou un chlore.

3. Utilisation selon l'une quelconque des revendications 1 ou 2, dans laquelle le substituant R est en position o.

4. Utilisation selon l'une quelconque des revendications 1 à 3, dans laquelle la dermatite est la dermatite atopique.

5. Utilisation selon l'une quelconque des revendications 1 à 4, dans laquelle le médicament est à usage externe.